# EUROPÄISCHE PATENTANMELDUNG

(11) **EP 0 709 060 A1**
(43) Veröffentlichungstag der Anmeldung: **01.05.1996**
(21) Anmeldenummer: 95112489.0
(22) Anmeldetag: 09.08.1995
(51) Int. Cl.: A61B 10/00

(54) **Biopsiegerät**

(30) Priorität: 27.10.1994 DE 4438333
(71) Anmelder: B. BRAUN MELSUNGEN AG, D-34212 Melsungen (DE)
(72) Erfinder: Orth, Manfred, Ing., D-34121 Kassel (DE); Woehr, Kevin Philip, Dipl.-Ing., D-34587 Felsberg (DE); Pape-Wehrlich, Wolfgang, D-37073 Göttingen (DE)
(74) Vertreter: Selting, Günther

(57) **Zusammenfassung**

Das Biopsiegerät weist ein langgestrecktes Gehäuse (210) auf, in dem ein Kanülenhalter (221) und ein Mandrinhalter (225) verschiebbar sind. Das Vortreiben des Kanülenhalters (221) erfolgt durch eine Federvorrichtung (233), wobei über eine Stanzlänge SL zunächst nur der Kanülenhalter (221) vorgeschoben wird. Anschließend wird über eine Kopplungseinrichtung (235) auch der Mandrinhalter (225) mitgenommen, wobei ein Nachschlag erfolgt, bei dem kein Gewebe mehr gestanzt, sondern nur noch zur Seite geschoben und abgeschert wird.

## Beschreibung

Die Erfindung betrifft ein Biopsiegerät zur Entnahme von Gewebeproben aus Körperorganen und -regionen.

Aus WO 92/19170 ist ein Biopsiegerät bekannt, das mit einer Kanüle und einem darin verschiebbaren Mandrin ausgestattet ist. Die Kanüle ist mit einem Kanülenkolben verbunden und der Mandrin mit einem Mandrinkolben. Beide Kolben sind in einem Gehäuse verschiebbar und der Kanülenkolben ist der Wirkung einer Feder ausgesetzt, die beim Auslösen des Gerätes die Kanüle vortreibt. Gleichzeitig wird der Mandrinkolben zurückbewegt, so daß Kanüle und Mandrin bei Stanzen gegenläufige Bewegungen ausführen, wodurch der vordere Kanülenbereich leer wird und stanzend in das Gewebe eindringt. Nach Beendigung des Stanzvorgangs erfolgt ein Nachschlag, bei dem Kanüle und Mandrin gemeinsam weiter in das Gewebe vordringen, um das Gewebe abzuscheren und zu verdichten und eine klar abgegrenzte Probenmenge im vorderen Endabschnitt der Kanüle zu erhalten. Das bekannte Biopsiegerät hat einen komplizierten Aufbau und benötigt zahlreiche Federn. Kanüle und Mandrin sind Bestandteil des Biopsiegeräts und können nicht ohne Demontage des Gerätes ausgewechselt werden.

Der Erfindung liegt die Aufgabe zugrunde, ein Biopsiegerät zu schaffen, das einen einfachen Aufbau hat, einfach zu bedienen ist, histopathologisch gut verwertbare Biopsate liefert und mit Wechselkanülen bedient werden kann.

Die Lösung dieser Aufgabe erfolgt erfindungsgemäß mit den im Patentanspruch 1 angegebenen Merkmalen.

Bei dem erfindungsgemäßen Biopsiegerät bewirkt eine einzige Federvorrichtung sowohl das Vorschieben des Kanülenhalters als auch die anschließende Mitnahme des Mandrinhalters, wobei der Bewegungsablauf in zwei Schritten erfolgt, nämlich dem Stanzen und dem Nachschlag. Beim Stanzen wird die Kanüle allein vorbewegt, während der Mandrin zurückbleibt. Dadurch nimmt der leere vordere Endbereich der Kanüle Gewebe auf. Beim anschließenden Nachschlag werden Kanüle und Mandrin in gleicher Weise weiter vorgeschoben. Hierdurch wird das im vorderen Kanülenbereich befindliche Gewebe nicht mehr gestanzt, sondern nur noch zur Seite geschoben und an der Schliffkante des Kanülenendes abgeschert. Wird das Biopsiegerät anschließend zurückgezogen, kann außerhalb des Körpers das Ausstoßen der Gewebeprobe aus der Kanüle erfolgen, indem der Mandrin innerhalb der Kanüle manuell vorgeschoben wird.

Eine Besonderheit der Erfindung besteht darin, daß das Stanzen und der Nachschlag in einem Zuge sehr schnell hintereinander ausgeführt werden. Hierdurch entsteht eine geringe Gewebetraumatisierung und es werden histopathologisch gut verwertbare Biopsate erzielt.

Ein weiterer Aspekt der Erfindung besteht darin, daß das aus der Kanüle und dem Mandrin bestehende Biopsiebesteck leicht ausgewechselt werden kann. Das Biopsiegerät bildet einen wiederverwendbaren Biopsieautomaten, wobei nur das Biopsiebesteck aus Wechselteilen besteht. Das Biopsiebesteck kann im zusammengeschobenen Zustand, also wenn der Mandrin vollständig in die Kanüle eingeschoben ist, in das Biopsiegerät eingesetzt werden, das am Kanülenhalter und am Mandrinhalter entsprechende Kopplungsmittel aufweist, mit denen die Ansatzstücke von Kanüle und Mandrin zusammenwirken.

Ein besonderer Vorteil ergibt sich, wenn die Kopplungseinrichtung einen verstellbaren Anschlag zur Veränderung der Stanzlänge aufweist. Durch Verstellen des Anschlags kann somit die Stanzlänge eingestellt werden. Damit kann die zu entnehmende Gewebemenge variiert werden. Bei Vergrößerung der Stanzlänge wird die Länge des Nachschlags nicht geändert.

Im folgenden werden unter Bezugnahme auf die Zeichnungen Ausführungsbeispiele der Erfindung näher erläutert.

Es zeigen:
- Fig. 1: einen schematischen horizontalen Längsschnitt durch ein erstes Ausführungsbeispiel des Biopsiegeräts,
- Fig. 2: einen vertikalen Teil-Längsschnitt durch Fig. 1,
- Fig. 3: eine Draufsicht des Biopsiebesteckes aus Kanüle und Mandrin,
- Fig. 4: in gleicher Darstellung wie Fig. 1 den Zustand des Biopsiegerätes bei Beendigung des Nachschlags,
- Fig. 5: die verschiedenen Zustände a) punktieren, b) Stanzen, c) Nachschlag und d) Ausstoßen der Gewebeprobe,
- Fig. 6: einen schematischen horizontalen Längsschnitt durch ein zweites Ausführungsbeispiel des Biopsiegeräts,
- Fig. 7: eine Seitenansicht der Ausführungsform von Fig. 6,
- Fig. 8: das Biopsiegerät nach Fig. 6 in der Endphase des Biopsievorganges,
- Fig. 9: in vergrößerter Darstellung den rückwärtigen Teil des Biopsiegeräts im gespannten Zustand,
- Fig. 10: den rückwärtigen Teil des Biopsiegerätes während des Stanzvorganges,
- Fig. 11: den rückwärtigen Teil des Biopsiegerätes im Anschluß an den Nachschlag während des Zurückziehens des Mandrins,
- Fig. 12: einen Schnitt entlang der Linie XII-XII von Fig. 9,
- Fig. 13: einen schematischen horizontalen Längsschnitt durch ein drittes Ausführungsbeispiel des Biopsiegerätes,
- Fig. 14: eine Seitenansicht des Biopsiegerätes nach Fig. 13,
- Fig. 15: in vergrößerter Darstellung den rückwärtigen Teil des Biopsiegerätes nach Fig. 13 in gespanntem Zustand,
- Fig. 16: einen Schnitt entlang der Linie XV-XV von Fig. 14.,
- Fig. 17: den rückwärtigen Teil des Biopsiegerätes kurz nach Beendigung des Stanzvorganges zu Beginn des Nachschlagens,
- Fig. 18: den rückwärtigen Teil des Biopsiegerätes am Ende des Nachschlags, und
- Fig. 19: einen Längsschnitt durch das Biopsiebesteck.

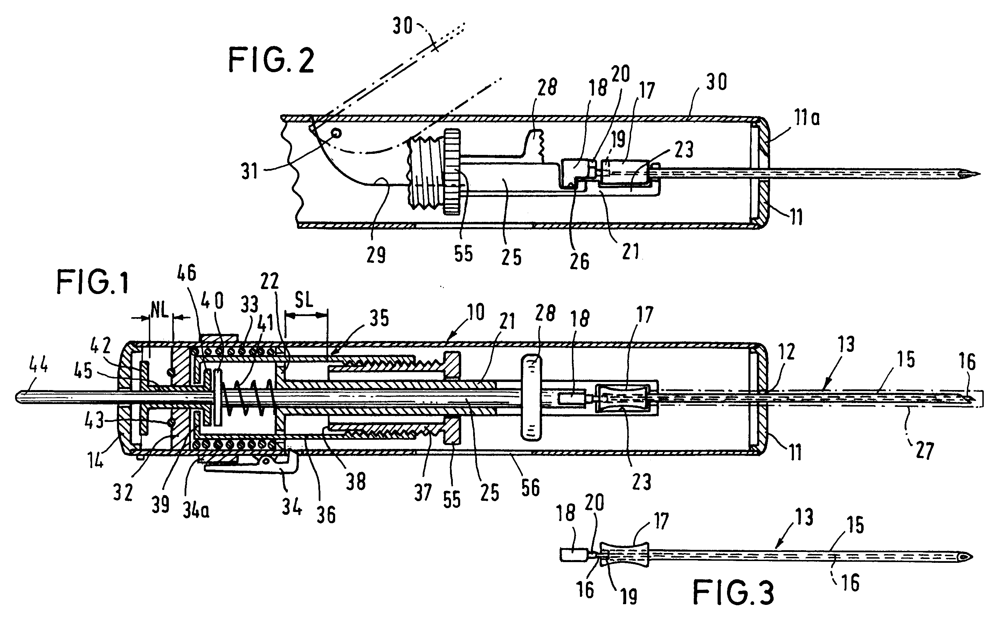

Das Biopsiegerät der Figuren 1 bis 4 weist ein langgestrecktes rohrförmiges Gehäuse 10 auf, das am vorderen Ende durch eine Frontwand 11 verschlossen ist, die eine Öffnung 12 für den Durchgang des Biopsiebestecks 13 aufweist. Am rückwärtigen Ende ist das Gehäuse 10 durch eine Rückwand 14 verschlossen.

Das Biopsiebesteck 13 besteht gemäß Fig. 3 aus der rohrförmigen Kanüle 15 und dem in die Kanüle 15 eingeführten Mandrin 16. Am rückwärtigen Ende der Kanüle 15 befindet sich ein aus Kunststoff bestehendes Ansatzstück 17 und am rückwärtigen Ende des Mandrins 16 befindet sich ein Ansatzstück 18. Das Ansatzstück 17 ist mit einer Kerbe 19 versehen, in die ein Vorsprung 20 des Ansatzstücks 18 eindringt, wenn der Mandrin vollständig in die Kanüle eingeschoben ist, um sicherzustellen, daß der Mandrin 16 stets in einer definierten Drehposition in der Kanüle enthalten ist.

In dem Gehäuse 10 ist ein Kanülenhalter 21 in Längsrichtung verschiebbar. Dieser Kanülenhalter besteht aus einer langgestreckten hohlen Stange, die am rückwärtigen Ende einen Flansch 22 aufweist. Am vorderen Ende des Kanülenhalters 21 befindet sich ein Kopplungsmittel 23 in Form einer Ausnehmung, in die das Ansatzstück 17 der Kanüle 15 passend eingesetzt werden kann.

Durch den Kanülenhalter 21 erstreckt sich ein Längsrichtung der Mandrinhalter 25 hindurch. Der Mandrinhalter 25 besteht aus einem langgestreckten Stab, der am vorderen Ende ein Kopplungsmittel 26 in Form einer Vertiefung aufweist, in die das Ansatzstück 18 des Mandrins 16 gemäß Fig. 2 passend einsetzbar ist. Im gespannten (geladenen) Zustand des Biopsiegerätes gemäß Fig. 1 haben die Kopplungsmittel 23 und 26 einen solchen gegenseitigen Abstand, daß die Ansatzstücke 17 und 18 des Biopsiebestecks im zusammengefügten Zustand in die Kopplungsmittel eingelegt werden können. Ferner ist das Biopsiebesteck 13 mit einer aus Kunststoff bestehenden Schutzhülse 27 versehen (Fig. 1), die die Kanüle 15 umgibt und auf das Ansatzstück 17 aufgeklemmt ist. Diese Schutzhülse 27 ragt durch die Öffnung 12 des Gehäuses hindurch. Sie wird erst abgezogen, nachdem das Biopsiebesteck 13 am Kanülenhalter und am Mandrinhalter befestigt worden ist.

An dem Kanülenhalter 21 ist in der Nähe des vorderen Endbereichs ein Spanngriff 28 vorgesehen, mit dem der Kanülenhalter zurückgeschoben werden kann, um das Gerät zu spannen. Der Spanngriff 28 befindet sich im Innern des Gehäuses 10. Das Gehäuse 10 ist an seiner Oberseite mit einer Öffnung 29 versehen, die mit einem Klappdeckel 30 verschlossen werden kann. Der Klappdeckel 30 ist um eine Achse 31 schwenkbar, die am rückwärtigen Ende der Öffnung 29 angeordnet ist. Der Klappdeckel wird also nach hinten aufgeklappt, wie dies in Fig. 2 gestrichelt dargestellt ist. Zum Klappdeckel 30 gehört auch ein Bereich 11a der Frontwand 11 des Gehäuses. Bei hochgeklapptem Klappdeckel 30 kann das Biopsiebesteck 13 in die entsprechenden Kopplungsmittel 23 und 26 eingesetzt werden.

Im rückwärtigen Bereich des Gehäuses 10 befindet sich eine feste Zwischenwand 32, an der die hier aus einer Schraubenfeder bestehende Federvorrichtung 33 für den Antrieb des Biopsiebestecks abgestützt ist. Das vordere Ende der Federvorrichtung 33 drückt gegen den Flansch 22 des Kanülenhalters 21. An diesem Flansch 22 greift auch eine Rückhaltevorrichtung 34 in Form eines Klinkenhebels an, die den Kanülenhalter 21 im gespannten Zustand festhält. Die Rückhaltevorrichtung 34 wird durch einen manuell verschiebbaren Sicherungsring 34a gesichert, der zurückgeschoben werden kann, um danach die Rückhaltevorrichtung 34 manuell auszulösen, so daß sie den Kanülenhalter 21 freigibt.

Der Mandrinhalter 25 wird von der selben Federvorrichtung 33 angetrieben, die auch den Kanülenhalter 21 antreibt. Hierzu ist der Mandrinhalter 25 mit dem Kanülenhalter 21 durch die Kopplungseinrichtung 35 gekoppelt. Diese Kopplungseinrichtung 35 weist ein Zugteil 36 in Form eines aus parallelen Stangen bestehenden Käfigs und ein Anschlagteil 37 auf. Das Anschlagteil 37 besteht aus einer Gewindebuchse, die in einen Gewindering des Zugteils 36 eingeschraubt ist. Am rückwärtigen Ende der Gewindebuchse ist ein Anschlag 38 gebildet, gegen den der Flansch 22 des Kanülenhalters 21 stoßen kann. Der Abstand des Anschlags 38 von dem Flansch 22 bildet die Stanzlänge SL. Nachdem der Kanülenhalter 21 die Stanzlänge durchlaufen hat, nimmt er den Mandrinhalter 25 mit, um den Nachschlag auszuführen. Die Mitnahme erfolgt über die Kopplungseinrichtung 35, deren Zugteil 36 mit einem nach innen gerichteten Ansatz 39 ein flanschartiges Mitnahmeteil 40 des Mandrinhalters 25 hintergreift. Zwischen dem Flansch 22 des Kanülenhalters 21 und dem Mitnahmeteil 40 des Mandrinhalters 25 befindet sich eine Federvorrichtung 41, die beim Ausstoßen der Gewebeprobe aus der Kanüle kompromiert wird und den Mandrin zur Startposition zurücktreibt.

Die Feder 41 stoppt auch den Mandrinhalter 25 am Ende des Nachschlags.

Das Vortreiben des Mandrinhalters 25 wird dadurch begrenzt, daß ein Flansch 42 gegen einen Endanschlag 43 stößt, der hier aus einem Gummiring besteht, welcher an der Rückseite der Zwischenwand 32 angebracht ist. Der Flansch 42 ist am rückwärtigen Ende einer durch die Zwischenwand 32 hindurchgehenden verschiebbaren Hülse 45 vorgesehen. Die Hülse 45 weist einen vorderen Flansch 46 auf, der von dem Ansatz 39 des Zugteils 36 hintergriffen wird und an dem Mitnahmeteil 40 anliegt. Bei vorgespanntem bzw. geladenem Instrument gibt der Abstand des Flansches 42 von dem Endanschlag 43 die Nachschlaglänge NL an, über die sich der Mandrinhalter 25 gemeinsam mit dem Kanülenhalter 21 bewegt. Der Mandrinhalter 25 erstreckt sich durch eine Mittelöffnung der Zwischenwand 32 hindurch. Am rückwärtigen Ende ist er mit einem Handgriff 44 versehen, der dazu dient, den Mandrinhalter 25 entgegen der Wirkung der Feder 41 vorzuschieben und dadurch die Gewebeprobe aus der Kanüle herauszustoßen.

Die Veränderung der Stanzlänge SL erfolgt durch Drehen des Anschlagteils 37 der Kopplungsvorrichtung 35. Zu diesem Zweck ist das Anschlagteil 37 mit einem Griffrad 55 versehen, das bei geöffnetem Klappdeckel 30 zugänglich ist und gedreht werden kann, um die Position des Anschlags 38 zu verändern. Das Griffrad 55 befindet sich im Bereich eines Fensters 56 des Gehäuses, an dem eine Skala angebracht sein kann, an der die eingestellte Stanzlänge SL abgelesen werden kann.

Die beschriebene Biopsievorrichtung arbeitet wie folgt:
Zunächst wird der Klappdeckel 30 geöffnet und durch Angreifen an dem Spanngriff 28 der Kanülenhalter 25 zurückgedrückt, wobei die Federvorrichtung 33 gespannt wird. Dann wird bei geöffnetem Klappdeckel das Biopsiebesteck 13 eingelegt, wobei das Ansatzstück 17 mit dem Kopplungsmittel 23 zusammengreift und das Ansatzstück 18 mit dem Kopplungsmittel 26. Dann wird der Klappdeckel 30 geschlossen und das Biopsiegerät ist betriebsbereit.

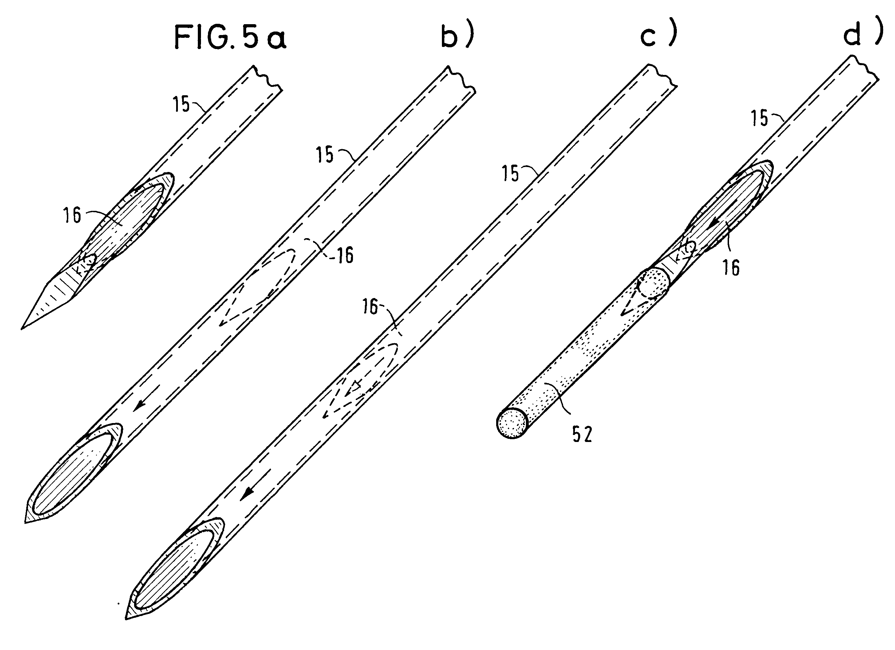

Vor Benutzung des Biopsiegeräts wird die Schutzhülse 27 aus dem Gerät herausgezogen, so daß die Kanüle 15 frei liegt. Aus der Kanüle 15 ragt der Mandrin 16 heraus, so wie dies in Fig. 5a) dargestellt ist.

In dem Zustand von Fig. 5a) erfolgt die Punktion, wobei das Biopsiebesteck durch die Haut und die nachfolgenden Schichten des Patienten bis zum Zielgewebe vorgeschoben wird. Dieses Vorschieben erfolgt zweckmäßigerweise unter Verwendung eines Ultraschallkopfes, durch dessen Öffnung das Biopsiebesteck 13 hindurchgeführt wird.

Wenn die Kanülenspitze in das Zielgewebe eingedrungen ist, wird der Sicherungsring 34a zurückgezogen und die Rückhaltevorrichtung 34 ausgelöst. Dabei wird der Kanülenhalter 21 durch die Federvorrichtung 33 vorgeschoben, während der Mandrinhalter 25 in seiner Position (in bezug auf das Gehäuse 10) verbleibt. Dies ist der Stanzvorgang gemäß Fig. 5b).

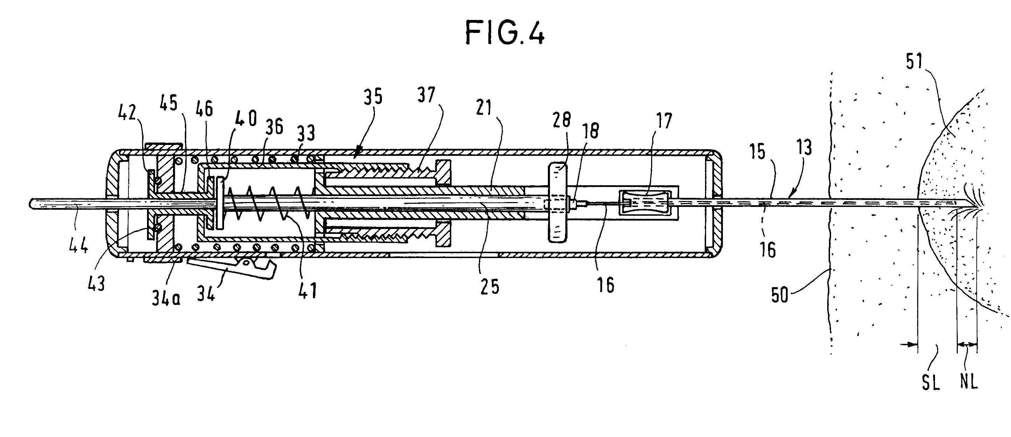

Wenn der Flansch 22 des Kanülenhalters 21 gegen den Anschlag 38 stößt, ist das Stanzen beendet. Der Kanülenhalter 21 wird jedoch übergangslos, d.h. ohne anzuhalten, von der Federvorrichtung weiter vorgeschoben und nimmt dabei über die Kopplungseinrichtung 35 den Mandrinhalter 25 mit. Das Biopsiebesteck führt hierbei gemaß Fig. 5c) den Nachschlag aus. Dieser Zustand ist auch in Fig. 4 dargestellt. Das Biopsiebesteck hat die Haut 50 durchdrungen und ist zunächst bis zum Zielgewebe 51 vorgeschoben worden. Dann wurde die Kanüle 15 um die Stanzlänge SL vorbewegt, und anschließend wurden Kanüle 15 und Mandrin 16 um die Nachschlaglänge NL gemeinsam bewegt, wobei die von der Kanüle aufgenommene Gewebeprobe von dem Zielgewebe 51 gelöst wurde.

Nachdem das Biopsiegerät zurückgezogen und dadurch das Biopsiebesteck aus dem Körper herausgezogen worden ist, wird durch Vorschieben des Handgriffs 44 der Mandrinhalter 25 noch weiter vorgeschoben, wobei gemäß Fig. 5d) der Mandrin 16 aus dem vorderen Ende der Kanüle 15 herausgeschoben wird und dabei die Gewebeprobe 52 ausstößt. Danach kann das Gerät neu gespannt und mit einem neuen Biopsiebesteck geladen werden.

Alternativ besteht die Möglichkeit, das Ausstoßen der Gewebeprobe dadurch zu bewirken, daß der Kanülenhalter 21 mit dem Handgriff 28 zurückgezogen wird, während der Mandrinhalter 25 nur denjenigen Teil der Rückzugsbewegung mitmacht, der der Nachschlaglänge NL entspricht. Hierbei würde das Ausstoßen der Gewebeprobe beim Zurückkehren in die Ausgangsposition gemäß Fig. 5a) erfolgen, wobei der Handgriff 44 und die Federvorrichtung 41 entbehrlich wären.

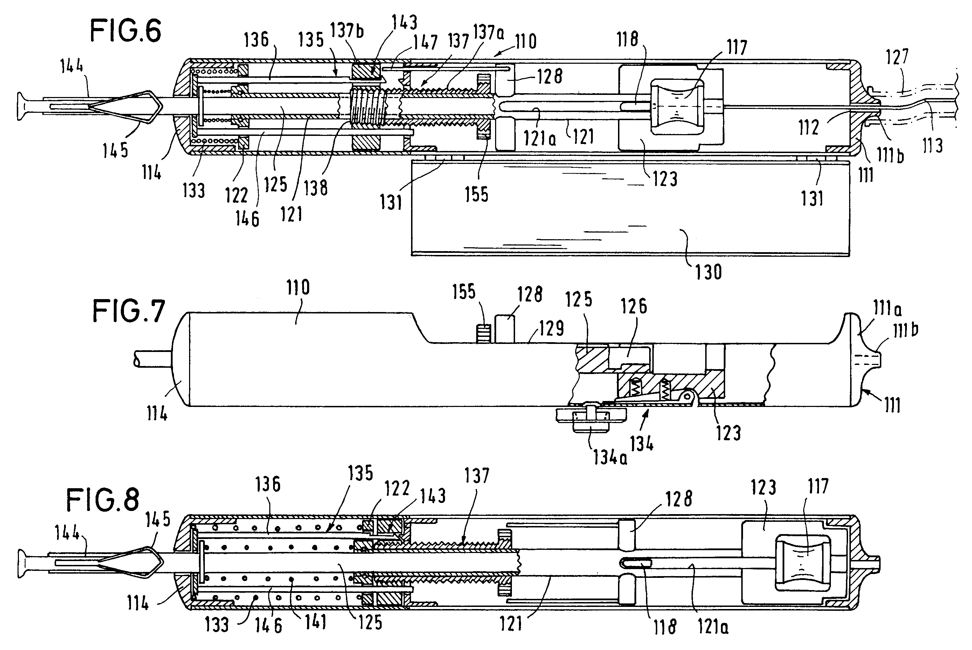

Im folgenden wird das Ausführungsbeispiel der Figuren 6 bis 12 beschrieben.

Das Biopsiegerät weist ein langgestrecktes rohrförmiges Gehäuse 110 auf, das am vorderen Ende durch eine Frontwand 111 verschlossen ist, in der sich eine Öffnung 112 für den Durchgang des Biopsiebestecks 113 befindet. Das Biopsiebesteck kann durch einen seitlichen Einführschlitz 111a in der Frontwand 111 in die Öffnung 112 seitlich eingeführt werden. Am rückwärtigen Ende ist das Gehäuse 110 durch eine Rückwand 114 verschlossen.

Das Biopsiebesteck 113 besteht, wie bei dem ersten Ausführungsbeispiel, aus einer rohrförmigen Kanüle und einem Mandrin. Am rückwärtigen Ende der Kanüle befindet sich ein Ansatzstück 117 und am rückwärtigen Ende des Mandrins befindet sich ein Ansatzstück 118.

In dem Gehäuse 110 ist ein Kanülenhalter 121 in Längsrichtung verschiebbar, der aus einer langgestreckten hohlen Stange besteht, die am rückwärtigen Ende einen fest angebrachten, radial abstehenden Flansch 122 aufweist. Am vorderen Ende des Kanülenhalters 121 befindet sich ein Kopplungsmittel 123 in Form einer Ausnehmung, in die das Ansatzstück 117 der Kanüle passend eingesetzt werden kann.

Durch den Kanülenhalter 121 erstreckt sich in Längsrichtung der Mandrinhalter 125 hindurch. Der Mandrinhalter besteht aus einem langgestreckten Stab, der am vorderen Ende ein Kopplungsmittel 126 in Form einer Vertiefung aufweist, in die das Ansatzstück 118 des Mandrins passend einsetzbar ist. Der hülsenförmige Kanülenhalter 121 weist einen Längsschlitz 121a auf, durch den das Ansatzstück 118 hindurchragt und der es ermöglicht, daß der Kanülenhalter vorgetrieben wird, während der Mandrinhalter mit dem Ansatzstück 118 stehenbleibt.

Das Biopsiebesteck 113 ist mit einer Schutzhülse 127 versehen, die auf einen konischen Ansatz 111b der Frontwand 111 aufgesteckt werden kann, um den aus dem Biopsiegerät herausragenden Teil des Biopsiebestecks 113 zu umgeben und zu schützen.

An dem Kanülenhalter 121 ist ein Spanngriff 128 vorgesehen, mit dem der Kanülenhalter zurückgeschoben werden kann, um das Gerät zu spannen. Der Spanngriff 128 befindet sich im Innern des Gehäuses 110. Das Gehäuse ist an seiner Oberseite mit einer Öffnung 129 versehen, die mit einem Klappdeckel 130 verschlossen werden kann. Der Klappdeckel 130 ist mit einem längslaufenden Seitenrand der Öffnung 129 über Gelenke 131 verbunden, so daß er um eine parallel zur Längsachse des Gehäuses 110 verlaufende Schwenkachse schwenkbar ist. Der Klappdeckel wird also seitlich aufgeschwenkt, wie dies aus Fig. 6 erkennbar ist. Bei aufgeklapptem Klappdeckel 130 kann das Biopsiebesteck 113 in die entsprechenden Kopplungsmittel 123 und 126 eingesetzt werden.

Die Federvorrichtung 133 für den Antrieb des Biopsiebestecks 113 ist an der Rückwand 114 des Gehäuses abgestützt. Das vordere Ende der Federvorrichtung 133 drückt gegen den Flansch 122 des Kanülenhalters 121.

In Fig. 6 ist die Federvorrichtung 133 mit dem Kanülenhalter 121 im gespannten Zustand dargestellt. In diesem gespannten Zustand wird der Kanülenhalter 121 durch die in Fig. 7 dargestellte Rückhaltevorrichtung 134 festgehalten, die mit dem Kopplungsmittel 123 verbunden ist, und am Gehäuse 110 angreift. Die Rückhaltevorrichtung 134 rastet beim Spannen durch Zurückschieben des Kanülenhalters 121 selbsttätig ein. Ein manuell betätigbarer Auslöser 134a kann gedrückt werden, um den Kanülenhalter freizugeben. Der Auslöser sichert sich beim Spannen des Gerätes automatisch, wenn der Auslöser 134a nach hinten geschoben wird. Um ihn zu entsichern, muß der Anwender den Auslöser 134a nach vorn schieben.

Der Mandrinhalter 125 wird von derselben Federvorrichtung 133 angetrieben, die auch den Kanülenhalter 121 vortreibt. Hierzu ist der Mandrinhalter 125 mit dem Kanülenhalter 121 durch die Kopplungseinrichtung 135 gekoppelt. Die Kopplungseinrichtung 135 weist ein Zugteil 136 in Form eines aus parallelen Stangen bestehenden Käfigs sowie ein Anschlagteil 137 auf, das aus einer Gewindehülse 137a mit Außengewinde und einer Mutter 137b mit Innengewinde besteht. Die Gewindehülse 137a ist in die Mutter 137b eingeschraubt und ihr rückwärtiges Ende bildet einen Anschlag 138 für den Flansch 122 des Kanülenhalters 121. Zur Einstellung der Position des Anschlags 138 ist die Gewindehülse 137a an ihrem vorderen Ende mit einem Griffrad 155 versehen. Durch die Gewindehülse 137a erstreckt sich der rohrförmige Kanülenhalter 121 hindurch.

Die Kopplungseinrichtung 135 enthält eine auslösbare Rastvorrichtung 143, die zwei Stangen des Zugteils 136 mit der Mutter 137b verbindet. Diese sich gegenüberliegenden Stangen haben Einkerbungen 143a, die in federndem Eingriff mit der Mutter 137b stehen. Wenn die Einkerbungen 143a die Mutter 137b verlassen, ist das Zugteil 136 von der Mutter 137b getrennt. Die Stangen des Zugteils 136, die mit den Kerben 143a versehen sind, ragen durch Längsbohrungen der Mutter 137b hindurch und sie stehen aus dem vorderen Ende der Mutter heraus. Die abgeschrägten Enden 143c können gegen eine Schräge 132a der Anschlagwand 132 stoßen, die im Gehäuse 110 fest angeordnet ist. Wenn dies geschieht, wird die Rastvorrichtung 143 gelöst. Das Zugteil 136 weist außerdem längere Stangen 146 auf, die durch Längsbohrungen der Mutter 137b und der Anschlagwand 132 hindurchragen und als Verdrehsicherung für das Zugteil 136 wirken.

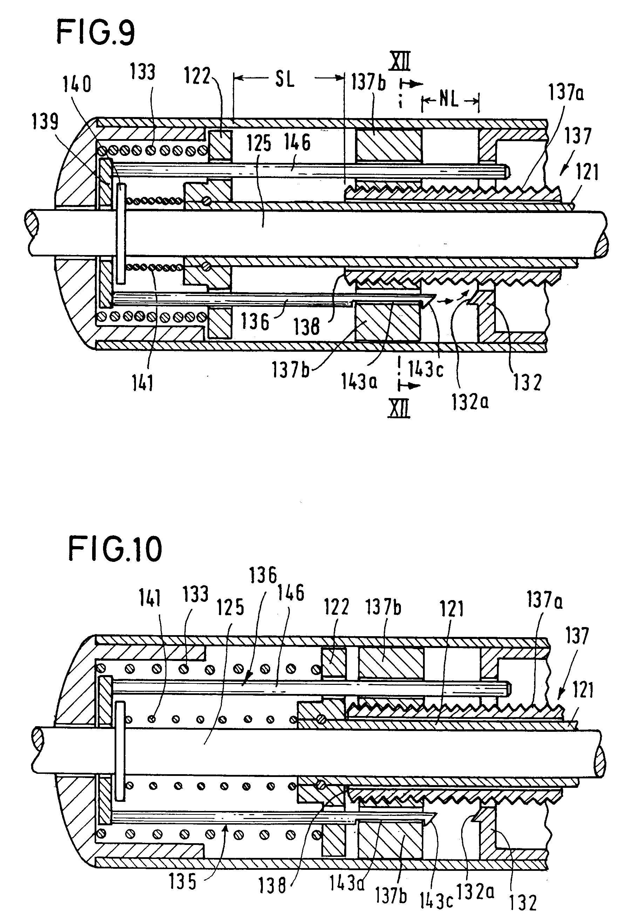

Der Abstand des Anschlags 138 von dem Flansch 122 bildet gemäß Fig. 9 die Stanzlänge SL. Nachdem der Kanülenhalter 121 die Stanzlänge durchlaufen hat, nimmt er den Mandrinhalter 125 mit, um den Nachschlag auszuführen. Die Veränderung der Stanzlänge SL erfolgt durch Drehen der Gewindehülse 137a, wodurch die Position des Anschlags 138 in bezug auf die Mutter 137b verändert werden kann. Der Abstand zwischen dem vorderen Ende der Mutter 137b und der Anschlagwand 132 bei geladenem bzw. vorgespanntem Gerät gemäß Fig. 9 gibt die Nachschlaglänge NL an, über die sich der Mandrinhalter 125 gemeinsam mit dem Kanülenhalter 121 bewegt.

Beim Nachschlag erfolgt die Mitnahme des Mandrinhalters 125 über die Kopplungseinrichtung 135, deren Zugteil 136 mit einem nach innen gerichteten Ansatz 139 ein flanschartiges Mitnahmeteil 140 des Mandrinhalters 125 hintergreift. Zwischen dem Flansch 122 des Kanülenhalters 121 und dem Mitnahmeteil 140 des Mandrinhalters 125 befindet sich eine Federvorrichtung 141, die beim Nachschlag teilweise entspannt wird und im Anschluß an den Nachschlag den Mandrinhalter 125 zurücktreibt, um den Mandrin in der Kanüle zurückzubewegen und dadurch einen Sog zu erzeugen, der die Gewebeprobe in die Kanüle hineinzieht.

Der Mandrinhalter 125 ist an seinem rückwärtigen Ende mit einem Auswurfstift 144 versehen, der durch die Rückwand 114 des Gehäuses 110 hindurchragt. An diesem Handgriff 144 befindet sich eine Anschlagfeder 145, die gegen die Rückwand 114 stoßen kann und dadurch die Vortriebslänge des Mandrinhalters 125 begrenzt.

Die Vorrichtung nach dem zweiten Ausführungsbeispiel arbeitet wie folgt:
Zunächst wird der Klappdeckel 130 gemäß Fig. 6 geöffnet und durch Angreifen an dem Spanngriff 128 der Kanülenhalter 121 zurückgedrückt, wobei die Federvorrichtung 133 gespannt wird. Dann wird bei geöffnetem Klappdeckel das Biopsiebesteck 113 eingelegt, nachdem zuvor die Schutzhülse 127 von dem Biopsiebesteck abgenommen wurde. Dann wird der Klappdeckel 130 geschlossen und die Schutzhülse 127 über den aus dem Gehäuse 110 herausragenden Teil des Biopsiebestecks 113 geschoben und auf den vorderen Ansatz 111b des Gehäuses 110 gesteckt. Vor Benutzung des Biopsiegeräts wird die Schutzhülse 127 entfernt und dann erfolgt die Punktion.

Wenn die Kanülenspitze in das Zielgewebe eingedrungen ist, wird der Auslöser 134a (Fig. 7) betätigt. Dadurch wird der Kanülenhalter 121 freigegeben, so daß er unter der Wirkung der Federvorrichtung 133 im Gehäuse 110 vordringt, während der Mandrinhalter 125 gemäß Fig. 10 in seiner Position (in bezug auf das Gehäuse 110) verbleibt. Dies ist der Stanzvorgang.

Wenn der Flansch 122 des Kanülenhalters 121 gegen den Anschlag 138 stößt, ist das Stanzen beendet. Der Kanülenhalter 121 wird jedoch von der Federvorrichtung 133, unterstützt durch die Federvorrichtung 141, weiter vorgeschoben und nimmt dabei über die Kopplungseinrichtung 135 den Mandrinhalter 125 mit. Das Biopsiebesteck 113 führt hierbei den Nachschlag aus, bei dem die Kanüle und der Mandrin um die Nachschlaglänge NL gemeinsam bewegt werden.

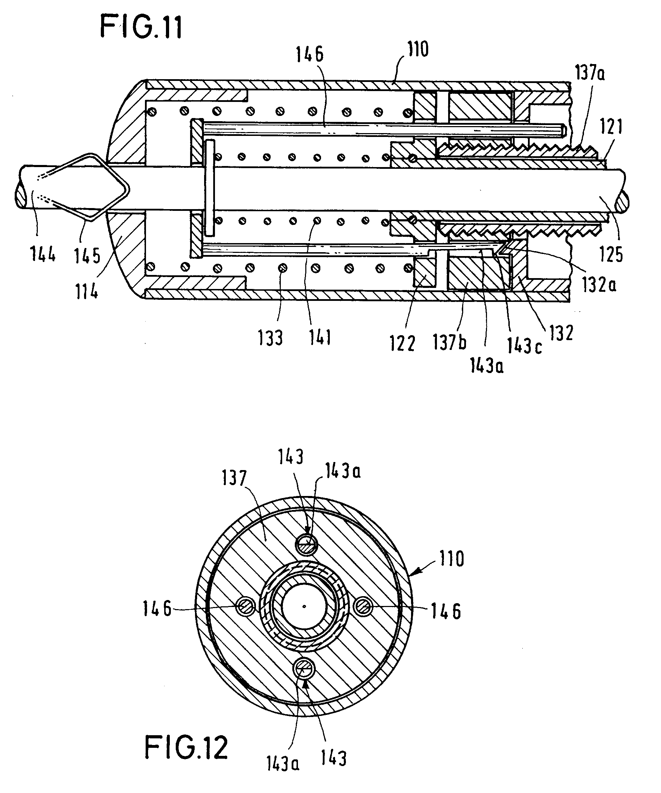

Am Ende des Nachschlags stoßen gemäß Fig. 11 die Enden 143c des Zugteils 136 gegen die Schräge 132a der Anschlagwand 132, wodurch die Rastvorrichtung 143, die das Zugteil 136 und die Mutter 137b zusammenhält, gelöst wird. Dadurch kann der Mandrinhalter 125 zusammen mit dem gelösten Zugteil 136 gemäß Fig. 11 von der Federvorrichtung 141 zurückbewegt werden. Durch dieses Zurückbewegen des Mandrins entsteht im vorderen Ende der Kanüle ein Sog oder Vakuum, wodurch die Gewebeprobe fest in die Kanüle hineingesaugt wird.

Nachdem das Biopsiegerät aus dem Körper herausgezogen worden ist, wird der Handgriff 144 vorgeschoben, um den Mandrin aus dem vorderen Ende der Kanüle herauszuschieben und dabei die Gewebeprobe auszustoßen. Dabei wird die Anschlagfeder 145 zusammengedrückt.

Beim darauffolgenden erneuten Spannen des Gerätes werden die Rastvorrichtungen 143 vom Stift 147 wieder in Eingriff gebracht.

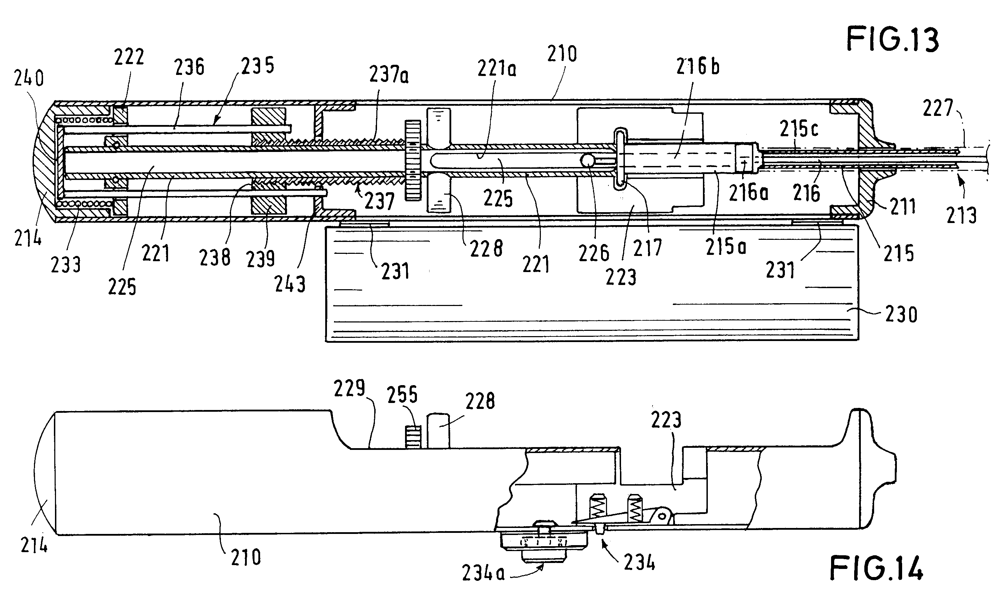

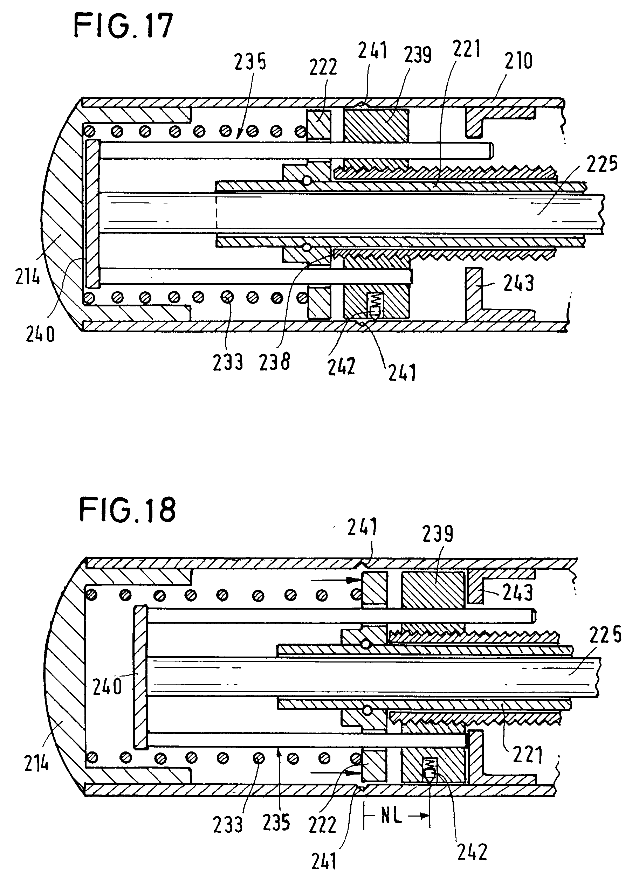

Im folgenden wird das Ausführungsbeispiel der Fign. 13 bis 19 beschrieben.

Das Biopsiegerät weist ein langgestrecktes rohrförmiges Gehäuse 210 auf, das am vorderen Ende durch eine Frontwand 211 verschlossen ist, in der sich eine Öffnung für den Durchgang des Biopsiebestecks 213 befindet. Das Biopsiebesteck kann durch einen seitlichen Einführschlitz in der Frontwand 211 eingeführt werden. Am rückwärtigen Ende ist das Gehäuse 210 durch eine geschlossene Rückwand 214 abgeschlossen.

Das Biopsiebesteck 213 ist in Fig. 19 gesondert dargestellt. Die Verwendung dieses Biopsiebestecks ist nicht auf die dargestellte Konstruktion eines Biopsiegerätes beschränkt. Vielmehr kann das Biopsiebesteck auch in Verbindung mit jedem beliebigen Biopsiegerät, also auch ohne Nachschlagbewegung, verwendet werden.

Das Biopsiebesteck 213 weist eine langgestreckte gerade Kanüle 215 auf, durch die sich ein Mandrin 216 erstreckt. Kanüle 215 und Mandrin 216 sind generell, wie bei dem ersten Ausführungsbeispiel ausgebildet. Das proximale Ende der Kanüle 215 ist mit dem distalen Ende eines Spritzenzylinders 215a verbunden. Am distalen Ende des Spritzenzylinders 215a ist ein Kanülenhalter 217 angeformt, mit dem der Spritzenzylinder und die Kanüle an dem Biopsiegerät fixiert werden können. Der Mandrin 216 erstreckt sich in den Spritzenzylinder 215a hinein. Er ist dort mit einem Kolben 216a verbunden, der in dem Spritzenzlyinder 215a in Längsrichtung verschiebbar ist und dessen Umfangsfläche gegen den Spritzenzylinder abdichtet. Von dem Kolben 216a erstreckt sich eine Kolbenstange 216b nach hinten durch das offene rückwärtige Ende des Spritzenzylinders 215a hindurch. Am proximalen Ende der Kolbenstange 216b ist ein Ansatzstück 218 vorgesehen, das mit dem Kopplungsmittel des Biopsiegerätes zusammengreift. Das Ansatzstück 218 weist eine Griffplatte 218a auf, sowie einen Ausleger 218b, der bei eingeschobenem Kolben 216a über das rückwärtige Ende des Spritzenzylinders 215a greift und sicherstellt, daß die Kolbenstange 216b sich in definierter Drehwinkellage im Spritzenzylinder befindet. Da der Mandrin 216 fest mit der Kolbenstange 216b verbunden ist, wird durch den Ausleger 218b erreicht, daß die Mandrinspitze eine definierte Winkellage zur Kanülenspitze einnimmt.

Wenn bei dem Stanzvorgang der Spritzenzylinder 215a zusammen mit der Kanüle 215 distal vorwärtsbewegt wird, während die Kolbenstange 216b mit dem Mandrin 216 festgehalten wird, entsteht im vorderen Bereich des Spritzenzylinders 215a ein Vakuum. Dieses Vakuum überträgt sich durch den Ringspalt zwischen Kanüle 215 und Mandrin 216 zum distalen Ende der Kanüle 215, das nunmehr über die Mandrinspitze hinausragt. Dadurch entsteht eine Saugkraft, die das Biopsat in das vordere Kanülenende hineinsaugt.

Gemäß Figur 13 ist in dem Gehäuse 210 ein Kanülenhalter 221 in Längsrichtung verschiebbar, der aus einer langgestreckten Stange besteht, die sich bis in die Nähe der Rückwand 214 des Gehäuses erstreckt. Am vorderen Ende des Kanülenhalters 221 befindet sich ein Kopplungsmittel 223, in das das Ansatzstück 217 am rückwärtigen Ende des Spritzenzylinders 215a eingesetzt wird. Das Kopplungsmittel 223 ist zusammen mit dem Kanülenhalter 221 in Gehäuselängsrichtung verschiebbar. Die Kanüle 215 ist von einer Schutzhülse 215c umgeben, welche abgezogen werden kann, nachdem das Biopsiebesteck 213 in das Biopsiegerät eingesetzt worden ist.

Durch den Kanülenhalter 221 erstreckt sich in Längsrichtung der Mandrinhalter 225 hindurch. Der Mandrinhalter besteht aus einem langgestreckten Stab, der am vorderen Ende ein Kopplungsmittel 226 zum Einsetzen des Ansatzes 218 des Biopsiebestecks 213 aufweist. Der hülsenförmige Kanülenhalter 221 weist einen Längsschlitz 221a auf, aus dem das Kopplungsmittel 226 herausragt und der es ermöglicht, daß der Kanülenhalter vorgetrieben wird, während der Mandrinhalter mit dem Ansatzstück 218 stehenbleibt.

An dem Kanülenhalter 221 ist ein Spanngriff 228 vorgesehen, mit dem der Kanülenhalter zurückgeschoben werden kann, um das Gerät zu spannen. Der Spanngriff 228 befindet sich im Innern des Gehäuses 210. Das Gehäuse ist an seiner Oberseite mit einer Öffnung 229 versehen (Fig. 14), die mit einem Klappdeckel 230 (Fig. 13) verschlossen werden kann. Der Klappdeckel 230 ist mit einem längslaufenden Seitenrand der Öffnung 229 über Gelenke 231 verbunden, so daß er um eine parallel zur Längsachse des Gehäuses 210 verlaufende Schwenkachse schwenkbar ist. Der Klappdeckel wird also seitlich aufgeschwenkt, wie dies aus Fig. 13 erkennbar ist. Bei aufgeklapptem Klappdeckel 230 kann das Biopsiebesteck 213 in die entsprechenden Kopplungsmittel 223 und 226 eingesetzt werden.

Die Federvorrichtung 233 für den Antrieb des Biopsiebestecks ist an der Rückwand 214 des Gehäuses abgestützt. Für sämtliche Bewegungen ist nur eine einzige Federvorrichtung 233 vorhanden, die aus einer oder mehreren Federn bestehen kann und nur auf den Kanülenhalter 221 einwirkt. Zu diesem Zweck ist in der Nähe des rückwärtigen Endes des Kanülenhalters 221 ein Mitnahmeteil 222 befestigt, gegen das die Federvorrichtung 233 drückt.

Im gespannten Zustand gemäß Fig. 13 wird der Kanülenhalter 221 durch die in Fig. 14 dargestellte Rückhaltevorrichtung 234 festgehalten, die mit dem Kopplungsmittel 223 verbunden ist und am Gehäuse 210 angreift. Die Rückhaltevorrichtung 234 rastet beim Spannen durch Zurückschieben des Kanülenhalters 221 selbsttätig ein. Ein manuell betätigbarer Auslöser 234a kann gedrückt werden, um den Kanülenhalter freizugeben. Der Auslöser sichert sich beim Spannen des Gerätes automatisch, wenn er nach hinten geschoben wird. Um ihn zu entsichern, muß der Anwender den Auslöser 234a nach vorne schieben.

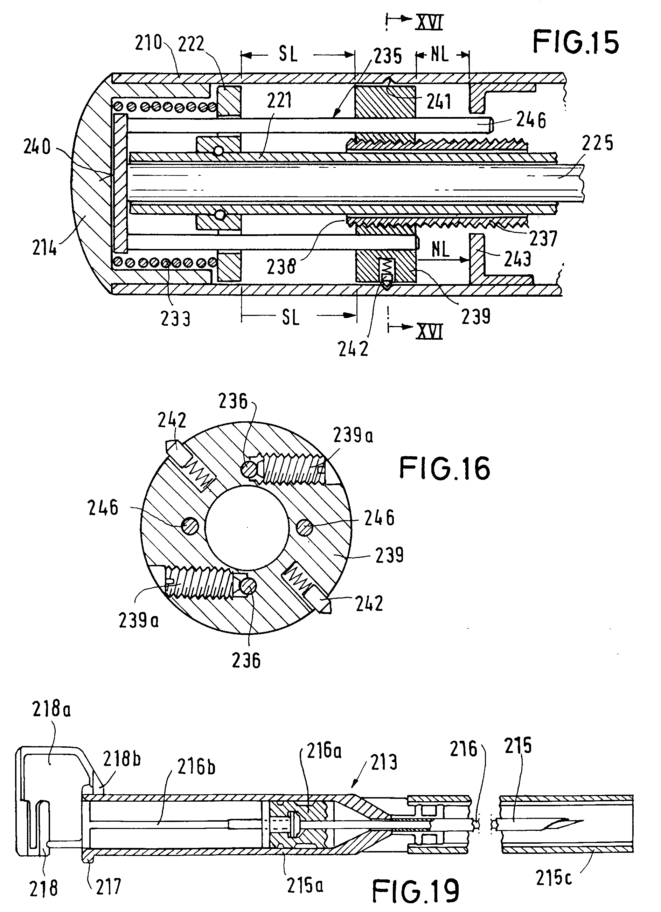

Der Mandrinhalter 225 wird von derselben Federvorrichtung 233 angetrieben, die auch den Kanülenhalter 221 vortreibt. Hierzu ist der Mandrinhalter 225 mit dem Kanülenhalter 221 durch die Kopplungseinrichtung 235 gekoppelt. Die Kopplungseinrichtung 235 weist einen Zugteil 236 in Form eines aus parallelen Stangen bestehenden Käfigs auf. Diese Stangen 236 sind am rückwärtigen Ende mit einem plattenförmigen Mitnahmeteil 240 verbunden, mit dem die rückwärtige stirnwand des Mandrinhalters 225 verbunden ist. Das Mitnahmeteil 240 bildet ferner eine Anschlagfläche beim Spannen für das rückwärtige Ende des Kanülenhalters 221. Die vorderen Enden der Zugteile 136 sind mit einer Scheibe 239 fest verbunden, die sich quer im Gehäuse erstreckt. Hierzu dienen gemäß Fig. 16 Fixierschrauben 239a, die in die Scheibe 239 eingeschraubt sind und gegen die Zugstangen 236 drücken.

Die Scheibe 236 ist ferner mit radial nach außen vorgespannten Rastelementen 242 versehen (Fig. 16), die in Rastausnehmungen 241 an der Innenseite des Gehäuses 210 eingreifen, um die Kopplungseinrichtung 235 in der Bereitschaftsstellung festzuhalten.

Die Kopplungseinrichtung 235 weist ferner ein verstellbares Anschlagteil 237 auf, dessen rückwärtiger Anschlag 238 in Bezug auf die Scheibe 239 einstellbar ist. Dieses Anschlagteil 237 besteht aus einer Gewindehülse, die mit einem Innengewinde der Scheibe 239 zusammengreift. Das Anschlagteil 237 ist mit einem Daumenrad 255 versehen, damit es manuell gedreht werden kann, um die Position des Anschlags 238 im Bereich der Kopplungseinrichtung 235 zu verändern.

Die Kopplungseinrichtung 235 weist ferner Verdrehsicherungsstäbe 246 auf, die über die Scheibe 239 hinaus vorstehen und in eine gehäusefeste Anschlagvorrichtung 243 hineinragen. Die Anschlagvorrichtung 243 dient gleichzeitig zur Begrenzung des Weges der Kopplungseinrichtung 235.

Der Abstand des Anschlagteils 222 von dem Anschlag 238 bildet gemäß Fig. 15 die Stanzlänge SL. Nachdem der Kanülenhalter 221 die Stanzlänge durchlaufen hat, nimmt er über die Kopplungseinrichtung 235 den Mandrinhalter 225 mit, um den Nachschlag auszuführen. Die Veränderung der Stanzlänge SL erfolgt durch Drehen der Gewindehülse 237a, wodurch die Position des Anschlags 238 in Bezug auf die Scheibe 239 verändert wird. Der Abstand zwischen dem vorderen Ende der Scheibe 239 in der Bereitschaftsstellung und der Anschlagwand 243 ist gemäß Fig. 9 die Nachschlaglänge NL, über die sich der Mandrinhalter 225 gemeinsam mit dem Kanülenhalter 221 bewegt.

Bei dem dritten Ausführungsbeispiel sind keine Teile vorhanden, die sich durch die Rückwand 214 erstrecken. Das Spannen des Gerätes erfolgt durch Zurückdrücken des Griffs 228, wobei zunächst der Kanülenhalter 221 zurückgeschoben wird. Dadurch, daß der Kanülenhalter gegen das Mitnahmeteil 240 der Kopplungseinrichtung 235 stoßt, wird auch die Kopplungseinrichtung und der Mandrinhalter 225 in die rückwärtige Endstellung geschoben, wobei die Rastelemente 242 in den Rastnuten 241 einrasten und der Mandrin das Biopsat aus der Kanüle treibt. Anstelle der in den Rastnuten einrastenden Rastelemente kann auch eine Reibbremse vorgesehen sein, die die Kopplungseinrichtung 235 während des Vorschnellens des Kanülenhalters 221 zunächst festhält und deren Kraft überwunden wird, wenn die Federvorrichtung 233 über das Mitnahmeteil 222 unmittelbar auf die Kopplungseinrichtung 235 einwirkt.

## Patentansprüche

1. Biopsiegerät mit
einem Gehäuse (10,110,210),
einem in Längsrichtung des Gehäuses (10,110,210) verschiebbaren Kanülenhalter (21,121,221) zur Befestigung des proximalen Endes einer Kanüle (15,115,215),
einem in Längsrichtung des Gehäuses (10,110,210) verschiebbaren Mandrinhalter (25,125,225) zur Befestigung des proximalen Endes eines durch die Kanüle (15,115,215) verlaufenden Mandrins (16,116,216),
einer an dem Gehäuse (10,110,210) abgestützten Federvorrichtung (33,133,233), die den Kanülenhalter (21,121,221) vortreibt,
einer auslösbaren Rückhaltevorrichtung (34,134,234) zum Festhalten des Kanülenhalters (21,121,221) bei gespannter Federvorrichtung (33,133,233) und
einer den Mandrinhalter (25,125,225) mit dem Kanülenhalter (21,121,221) koppelnden Kopplungseinrichtung (35,135,235), die bei der Vorwärtsbewegung des Kanülenhalters (21,121,221) nach Durchlaufen einer Stanzlänge (SL) den Mandrinhalter (25,125,225) über eine Nachschlaglänge (NL) mitnimmt.

2. Biopsiegerät nach Anspruch 1, dadurch gekennzeichnet, daß die Kopplungseinrichtung (35,135,235) einen verstellbaren Anschlag (38,138,238) zur Veränderung der Stanzlänge (SL) aufweist.

3. Biopsiegerät nach Anspruch 2, dadurch gekennzeichnet, daß die Kopplungseinrichtung (35,135,235) ein Zugteil (36,136,236) aufweist, das ein Mitnahmeteil (40,140,240) des Mandrinhalters (25,125,225) hintergreift und mit einem Anschlagteil (37,137,237) verbunden ist, gegen das bei Vorwärtsbewegen des Kanülenhalters (21,121,221) ein Mitnahmeteil (22,122,222) des Kanülenhalters (21,121,221) stößt.

4. Biopsiegerät nach einem der Ansprüche 1-3, dadurch gekennzeichnet, daß ein Endanschlag (43,143,243) vorgesehen ist, der den Vorschubweg des Mandrinhalters (25,125,225) und über die Kopplungseinrichtung (35,135,235) auch den Vorschubweg des Kanülenhalters (21,121,221) begrenzt.

5. Biopsiegerät nach einem der Ansprüche 1-4, dadurch gekennzeichnet, daß das Gehäuse (10,110,210) eine Öffnung (29,129,229) aufweist, durch die ein Griff (28,128,228) des Kanülenhalters (21,121,221) zum Zurückschieben und zum Spannen der Federvorrichtung (33,133,233) zugänglich ist.

6. Biopsiegerät nach Anspruch 5, dadurch gekennzeichnet, daß die Öffnung (29,129,229) mit einem Klappdeckel (30,130,230) verschließbar ist.

7. Biopsiegerät nach einem der Ansprüche 1-6, dadurch gekennzeichnet, daß der Kanülenhalter (21,121,221) und der Mandrinhalter (25,125,225) an ihren vorderen Enden Kopplungsmittel (23,26;123,126;223,226) zum Einsetzen der Ansatzstücke (17,18; 117,118;217,218) von Kanüle (15,115,215) und Mandrin (16,116,216) aufweisen.

8. Biopsiegerät nach einem der Ansprüche 1-7, dadurch gekennzeichnet, daß die Rückhaltevorrichtung (134,234) einen manuell betätigbaren Auslöser (134a,234a) aufweist, der beim Spannen durch Zurückschieben des Kanülenhalters (121,221) selbsttätig einrastet und sich sichert.

9. Biopsiegerät nach einem der Ansprüche 1-8, dadurch gekennzeichnet, daß die Kopplungseinrichtung (235) in einer Bereitschaftsposition rastend oder klemmend am Gehäuse (210) gehalten ist und nachdem der Kanülenhalter (221) die Stanzläge (SL) durchlaufen hat, über die Nachschlaglänge (NL) unter Überwindung der Rastung oder Klemmung mitgenommen wird.

10. Biopsiegerät nach einem der Ansprüche 1 bis 9, dadurch gekennzeichnet, daß für den Kanülenhalter (221) und den Mandrinhalter (225) eine einzige Federvorrichtung (233) vorhanden ist, die auf den Kanülenhalter (221) einwirkt und sich am Gehäuse (210) abstützt.

11. Biopsiegerät nach einem der Ansprüche 1-10, dadurch gekennzeichnet, daß die Rückwand (214) des Gehäuses (210) geschlossen ist.

12. Biopsiegerät nach einem der Ansprüche 1-11, dadurch gekennzeichnet, daß die Kopplungseinrichtung (235) eine - ggf. längenveränderbare - starre Einheit bildet.

13. Biopsiegerät nach einem der Ansprüche 1-12, dadurch gekennzeichnet, daß die Kopplungseinheit (235) ein Mitnahmeteil (240) aufweist, an dem die rückwärtigen Enden von Mandrinhalter (225) und Kanülenhalter (221) in der Bereitschaftsstellung anliegen.

14. Biopsiebesteck, insbesondere für ein Biopsiegerät nach einem der Ansprüche 1-13, mit einer Kanüle (215) und einem in Längsrichtung der Kanüle bewegbaren Mandrin (216), dadurch gekennzeichnet, daß die Kanüle (215) mit einem Spritzenzylinder (215a) und der Mandrin (216) mit einem in den Spritzenzylinder bewegbaren Kolben (216a) versehen ist, wobei der Kolben (216) beim Zurückziehen des Mandrins (216) in dem Spritzenzylinder (215a) einen Unterdruck erzeugt, der zwischen Kanüle (215) und Mandrin (216) bis zum distalen Kanülenende wirkt und ein darin befindliches Biopsat ansaugt.
